# EUROPEAN PATENT APPLICATION

(11) **EP 3 034 161 A1**
(43) Date of publication of application: **22.06.2016**
(21) Application number: 14198922.8
(22) Date of filing: 18.12.2014
(51) Int. Cl.: B01J 8/04, C07C 29/151, C07C 29/152, C07C 29/86, C07C 29/94, C07C 31/04

(54) **Method and reactor design for the production of methanol**

(71) Applicant: Haldor Topsøe A/S, 2800 Kgs. Lyngby (DK)
(72) Inventor: Modarresi, Hassan, 2800 Kgs. Lyngby (DK)
(74) Representative: Haldor Topsøe A/S

(57) **Abstract**

In a method for the production of methanol via exothermic equilibrium reactions, methanol, water and heat are partly removed from the methanol synthesis reactor or from a point between two or more methanol synthesis reactors by means of a solvent capable of absorbing methanol, water and heat. A preferred reactor design comprises a boiling water reactor R1 and a second methanol synthesis reactor R2. The method enables sufficient temperature control across the catalytic bed. The most preferred solvent is an alcohol, such as propylene glycol or glycerol.

## Description

The present invention relates to a novel method for the production of methanol and a reactor design for carrying out the method.

Methanol is produced from synthesis gas (syngas) via an equilibrium reaction, which proceeds at elevated temperature under elevated pressure. The synthesis reactions are:

CO + H₂O <-> CH₃OH + heat (1)

CO₂ + 3H₂ <-> CH₃OH + H₂O + heat (2)

CO + H₂O <-> CO₂ + H₂ + heat (3)

One of the problems associated with methanol production is the difficulty of sufficient temperature control across the catalytic bed. The conversion of syngas to methanol is extremely exothermic and causes a sharp temperature rise in the catalytic bed unless an appropriate means of heat removal is applied. If the reaction heat is not removed from the reactor, the temperature will rise to the maximum equilibrium temperature, which is usually as high as 300°C in a typical methanol synthesis reactor. Such a high temperature will not only generate a considerable amount of various by-products, but also deactivate the catalyst due to a sintering phenomenon.

Unconverted syngas is recovered from the methanol synthesis reactor and recycled back to the reactor. The amount of re-cycling depends on one-pass conversion, higher conversion, lower recycling and consequently smaller synthesis loop size as well as lower operating cost for the recycle compressor.

It is known that in situ withdrawal or post-withdrawal of heat and/or methanol and water from the synthesis reactor will enhance the conversion due to le Chatelier's principle, which states that if a dynamic equilibrium is disturbed by a change in conditions, then the position of equilibrium moves to counteract this change. According to the method of the present invention heat, methanol and water will be partly removed from the methanol synthesis reactor or from a point between two or more synthesis reactors, which is done by using a solvent capable of absorbing methanol, water and heat. The type and amount of solvent will determine the degree of conversion in a one pass reactor at a specific process condition.

The selection of the solvent is a key aspect of the present invention. More specifically, an appropriate solvent needs to be readily available, non-toxic, non-corrosive, cheap and thermally stable. In addition, the solvent must possess a very low vapour pressure, viscosity and syngas dissolving capacity. It needs to be a good solvent for methanol at an elevated pressure and temperature, but it should preferably have a low affinity for methanol dissolving at low temperatures, possibly leading to a liquid-liquid phase split in order to separate methanol from the solvent.

Furthermore, the solvent needs the ability to be readily recovered and purified from methanol and dissolved syngas for recycling. In addition, the solvent is preferably nonreactive with the catalyst in the methanol synthesis condition. If not, it must at least be neutral to the catalyst, thus causing no deactivation or poisoning.

Suitable solvents for use in the method according to the invention are propylene glycol and glycerol. Higher alcohols, such as n-decanol, can also be used. Furthermore, blends of different solvents may be used to enhance the performance of the wash unit. The method of the invention is however not limited to these solvents, which means that any solvent or blend of solvents, which can fulfil the process and safety requirements as well as the economic criteria, may be used.

CN 103274357 A discloses an adsorption enhanced method for steam reforming of methanol. Carbon dioxide in the product of the reforming reaction is adsorbed and removed through an adsorbent by utilizing le Chatelier's principle, so that the reforming reaction is promoted to move in a hydrogen generation direction. This way, the methanol and steam reforming reaction is reinforced, and the reaction temperature is reduced. Furthermore, because the side reaction of carbon monoxide is inhibited after the carbon dioxide concentration has been reduced, the purpose of radically reducing the carbon monoxide concentration is achieved. The CN publication further discloses a device for hydrogen production by methanol steam reforming, which is based on continuous adsorption reinforcement, said device comprising four parallel reactors and matching heating or decompressing equipment.

From US 2007/0244208 a process for producing high octane liquid fuel from carbon dioxide and water is known. The feedstock for the production line is industrial carbon dioxide and water, which may be of lower quality. The end product can be high octane gasoline, high cetane diesel or other liquid hydrocarbon mixtures suitable for further industrial processing or commercial use. Products such as dimethyl ether or methanol may also be withdrawn from the production line. The process is emission-free and able to reprocess all hydrocarbons, which are not suited for use as liquid fuels, to form high octane products. The heat, which is generated by exothermic reactions in the process, is fully utilized and so is the heat produced in the reprocessing of hydrocarbons, which are not suitable for liquid fuels.

However, both in the CN and in the US publication, reactions different from those of the present method are disclosed. Also the solvents/absorbents are different from those used in the method of the present invention, i.e. they are not alcohols, and the process steps are also different from those of the present method.

Thus, the novel method according to the invention is a method for the production of methanol via the exothermic equilibrium reactions (1)-(3), wherein methanol, water and heat are partly removed from the methanol synthesis reactor or from a point between two or more methanol synthesis reactors by means of a solvent capable of absorbing methanol, water and heat.

The solvent for use in the method according to the invention is preferably an alcohol. Most preferred alcohols are propylene glycol and glycerol. Table 1 compares the properties and performance of propylene glycol and glycerol with other potential solvents. In this table some key process parameters of various solvents in a wash unit are benchmarked against glycerol solvent. A wash unit comprises a high pressure counter-flow liquid-gas contactor, where fresh solvent along with recovered solvent is contacted with high temperature and pressure syngas with methanol content. The solvent absorbs methanol and partly other species, and it exits the contactor from the bottom. The lean gas (lean in methanol) leaves the contactor from the top. The effluent solvent is depressurized and degased first; then it is treated in a solvent recovery unit, such as a distillation unit, to separate the solvent from the methanol product. The solvent is pressurized via a pump and recycled to the contactor along with make-up fresh solvent to compensate solvent losses. According to this description, Sin in Table 1 refers to inlet solvent weight flow, and Sin/Gin indicates the weight ratio of the inlet solvent and the inlet gas to the contactor. Sout and Gout refer to outlet solvent and gas from the contactor, respectively. Solvent loss and gas loss are an indication of lost solvent in washed gas and trapped gas in the washing solvent, both in percentage of inlet solvent and inlet gas, respectively. From Table 1, it is seen that glycerol standing out as the best solvent among the listed solvents in terms of solvent loss, gas loss. Due to very low loss in solvent, required make-up solvent is also very low.

**Table 1**

| Solvent wash unit to recover 90% of methanol in gas stream entering the wash column at 257°C and 87.4 barg with mole composition (%) : CH₃OH 31.3, CO 10.2, CO₂ 8.4, H₂ 48.5, the balance being inerts and by-products | | | | | | |
|---|---|---|---|---|---|---|
| **solvent** | **Sin** | **Sin/Gin** | **solvent loss*** | **gas loss*** | **Sₒᵤₜ T** (°C) | **Gₒᵤₜ T** (°C) |
| Glycerol | 1.00 | 0.86 | 0.03 | 0.009 | 229 | 159 |
| Dipropylene glycol | 0.99 | 1.57 | 0.22 | 0.05 | 220 | 147 |
| Decanol | 1.11 | 1.31 | 0.24 | 0.05 | 217 | 147 |
| Benzyl alcohol | 1.16 | 1.18 | 0.65 | 0.22 | 226 | 157 |
| Propylene glycol | 0.86 | 1.56 | 1.00 | 0.36 | 226 | 161 |
| Dimethanol amine | 1.06 | 1.66 | 6.6 | 2.4 | 218 | 165 |
| Ethylene glycol | 1.06 | 1.45 | 6.6 | 2.4 | 218 | 165 |
| 1-Butanol | 0.92 | 1.66 | 11.57 | 4.4 | 215 | 169 |
| water | 0.54 | 1.35 | 16.60 | 15.7 | 206 | 200 |
| 1-Propanol | 0.84 | 1.83 | 22.54 | 9.1 | 208 | 173 |

| | | | | | | |
|---|---|---|---|---|---|---|
| Sin: relative inlet solvent weight base flow; Sin/Gin: solvent to gas weight ratio; *) in wt% | | | | | | |

The method according to the invention is highly beneficial in large methanol plants, whereas in a single train synthesis process, the reactor size would be a limiting factor. Pushing the reactions (1) and (2) to the right hand side in a single pass reactor would minimize the catalyst volume as well as the recycle ratio of the synthesis loop. Nevertheless, the process scheme is not limited to large production plants.

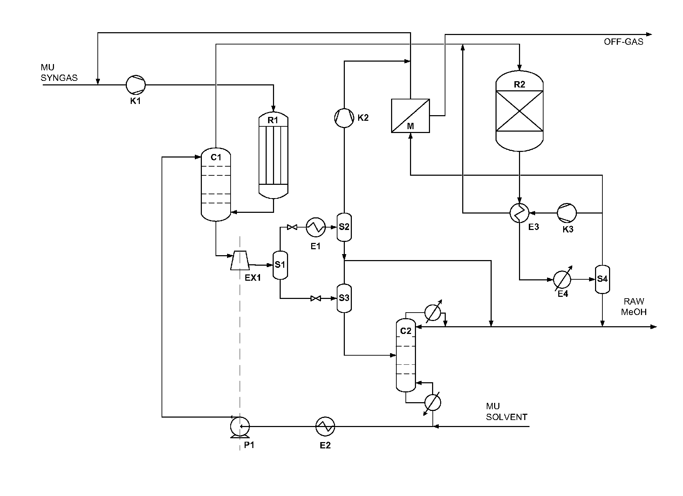

The method of the invention is illustrated in more detail in the following example with reference to the figure, which shows a suitable reactor design without being limited thereto.

### Example

The figure shows a reactor design comprising two reactors, i.e. a boiling water reactor R1 and a second methanol synthesis reactor R2. Make-up (MU) syngas is compressed together with recovered hydrogen from the synthesis loop off-gas and desorbed syngas from a wash unit, and the compressed mixture is fed to the boiling water reactor R1. The effluent from R1 at 260°C is brought into contact with the selected solvent in the wash unit C1 and cooled down to 180-190°C. The cooled, methanol-lean gas is fed to the second methanol synthesis reactor R2. The product stream from R2 is cooled and raw methanol is separated from the product stream, and unconverted syngas is recycled to reactor R2. A purge stream is sent to the membrane unit M. The recovered high concentration hydrogen stream is recycled to the make-up gas compressor K1.

Rich solvent from C1 is depressurized in the expander EX1 and passed through high pressure separator (S1), medium pressure separator (S2) and low pressure separator (S3) columns to recover solvent from dissolved methanol, water and syngas. Remaining methanol-water in the solvent is recovered in the distillation column C2. Pure solvent is mixed with make-up (MU) solvent, cooled and pressurized via the pump P1 and fed to the wash unit C1. Power recovered from the expander EX1 is in far excess relative to the power needed in the pump P1, which means that there is no need for an external power source for P1 during normal operation.

## Claims

1. A method for the production of methanol via the exothermic equilibrium reactions
CO + H₂O <-> CH₃OH + heat (1)
CO₂ + 3H₂ <-> CH₃OH + H₂O + heat (2)
CO + H₂O <-> CO₂ + H₂ + heat (3)
wherein methanol, water and heat are partly removed from the methanol synthesis reactor or from a point between two or more methanol synthesis reactors by means of a solvent capable of absorbing methanol, water and heat.

2. The method according to claim 1, wherein the solvent capable of absorbing methanol, water and heat is an alcohol.

3. The method according to claim 2, wherein the alcohol is either glycerol, propylene glycol or a mixture of both.

4. A reactor design for carrying out the method for production of methanol according to any of the claims 1-3, said reactor design comprising two reactors R1 and R2, where R1 is a boiling water reactor and R2 is a second methanol synthesis reactor.

5. The reactor design according to claim 4, further comprising a high pressure separator (S1) column, a medium pressure separator (S2) column and a low pressure separator (S3) column to recover solvent from dissolved methanol, water and syngas.

6. The reactor design according to claim 4 or 5, further comprising a wash unit C1, where the selected solvent is brought into contact with the effluent from the reactor R1, and a distillation column C2 for recovering remaining methanol-water in the solvent.
